# EUROPEAN PATENT SPECIFICATION

(11) **EP 4 226 647 B1**
(45) Date of publication and mention of the grant of the patent: **03.12.2025**
(21) Application number: 21878585.5
(22) Date of filing: 08.10.2021
(51) Int. Cl.: H04R 1/28, A61B 7/02, G01H 1/00, A61B 7/04, G10K 11/02, H04R 19/01, H04R 31/00, H04R 1/44, H04R 1/46

(54) **IMPEDANCE-MATCHED ACOUSTIC TRANSDUCER**
IMPEDANZANGEPASSTER AKUSTISCHER WANDLER
TRANSDUCTEUR ACOUSTIQUE À ADAPTATION D'IMPÉDANCE

(30) Priority: 09.10.2020 US 202063089702 P
(43) Date of publication of application: 16.08.2023
(73) Proprietor: The Johns Hopkins University, Baltimore, MD 21218 (US)
(72) Inventor: WEST, James, Edward, Baltimore, MD 21218 (US); RENNOLL, Valerie, Baltimore, MD 21218 (US); EISAPE, II, Adebayo, Ayoola, Baltimore, MD 21218 (US); MCLANE, Ian, Baltimore, MD 21218 (US); ELHILALI, Mounya, Baltimore, MD 21218 (US)
(74) Representative: London IP Ltd
(86) International application number: PCT/US2021/054088
(87) International publication number: WO 2022/076770

(56) References cited:
- WO-A1-2019/173470
- CN-A- 101 770 047
- CN-A- 109 115 376
- CN-A- 111 248 888
- CN-Y- 201 348 904
- TW-A- 200 729 994
- US-A1- 2009 316 925
- US-A1- 2011 249 834
- US-A1- 2015 173 625
- US-A1- 2016 155 433
- US-A1- 2018 367 884
- US-A1- 2019 133 553
- US-A1- 2019 321 007
- US-B2- 8 824 706
- MCLANE IAN M.: "Flexible electrostatic transducer with tuned acoustic impedance for improved sensing of body-and water-borne sounds", 1 October 2020 (2020-10-01), XP093125311, Retrieved from the Internet <URL:https://pubs.aip.org/asa/jasa/article/148/4_Supplement/2696/645266/Flexible-electrostatic-transducer-with-tuned> [retrieved on 20240130]
- RENNOLL VALERIE ET AL: "Electrostatic Acoustic Sensor with an Impedance-Matched Diaphragm Characterized for Body Sound Monitoring", vol. 6, no. 8, 20 July 2023 (2023-07-20), US, pages 3241 - 3256, XP093125610, ISSN: 2576-6422, Retrieved from the Internet <URL:https://pubs.acs.org/doi/pdf/10.1021/acsabm.3c00359> [retrieved on 20240130], DOI: 10.1021/acsabm.3c00359

## Description

### CROSS-REFERENCE TO RELATED APPLICATIONS

This application claims priority to U.S. Provisional Patent Application No. 63/089,702, filed on October 9, 2020.

### GOVERNMENT SUPPORT

This invention was made with government support under grants HL133043 and MD014104 awarded by the National Institutes of Health. The government has certain rights in the invention.

### FIELD OF THE INVENTION

The present invention relates generally to transducers. More particularly, the present invention relates to impedance-matched acoustic transducers.

### BACKGROUND OF THE INVENTION

We are constantly surrounded by an abundance of acoustic energy, whether from speech, music, or environmental noise. If captured, these acoustic waves can provide a wealth of information about a person and his/her surroundings, and this information be leveraged for multiple applications. In a first example, body sounds may provide health and diagnostic details. In a second example, sound underwater may be used for navigation and tracking. In a third example, vibrations from physical objects may provide details related to structural monitoring and harvesting energy to/for power small devices.

Currently, there are a variety of acoustic transducers (e.g., electrodynamic, electromagnetic, electrostatic, and piezoelectric) that can be used for these applications depending on the frequency range of interest, required sensitivity, and medium being monitored. Despite this wide selection, a problem that plagues these transducers is the acoustic impedance mismatch between the device and media being monitored, which results in lowered energy density at the transducer element and interference from other sound sources. MclaneIan "Flexible electrostatic transducer with tuned acoustic impedance for improved sensing of body- and water-borne sounds", published on 1 October 2020 discloses metal-coated microstructures on the surface of an elastomer that create small gaps with the charged polymer electret film.

### SUMMARY OF THE INVENTION

An acoustic transducer is disclosed according to claim 1. A method comprising building an acoustic transducer is disclosed according to claim 14. Advantageous embodiments are defined in the dependent claims.

### BRIEF DESCRIPTION OF THE DRAWINGS

The accompanying drawings provide visual representations, which will be used to more fully describe the representative embodiments disclosed herein and can be used by those skilled in the art to better understand them and their inherent advantages. In these drawings, like reference numerals identify corresponding elements and:
Figure 1 illustrates a schematic cross-sectional view of a transducer, according to an embodiment.
Figure 2 illustrates a graph showing a range of impedance values that can be obtained by a first layer of the transducer, according to an embodiment.
Figure 3A illustrates a graph showing the acoustic output of the transducer with no noise, Figure 3B illustrates a graph showing the acoustic output of the transducer with noise, Figure 3C illustrates a graph showing the acoustic output of an ambient microphone with no noise, and Figure 3D illustrates a graph showing the acoustic output of the ambient microphone with noise, according to an embodiment.
Figures 4A-4C illustrate graphs showing a stability of a third layer of the transducer, according to an embodiment.
Figure 5 illustrates a graph showing examples of the voltage response of the transducer for a 1 Hz applied force, according to an embodiment.
Figure 6 illustrates a graph showing the voltage response of the transducer as a function of frequency, according to an embodiment.
Figure 7 illustrates a schematic cross-sectional view of another transducer, according to an embodiment.
Figure 8 illustrates a flowchart of a method for monitoring a medium, according to an embodiment.
1 lbs corresponds to 0.45359237 kg.

### DETAILED DESCRIPTION

The presently disclosed subject matter now will be described more fully hereinafter with reference to the accompanying Drawings, in which some, but not all embodiments of the inventions are shown. Like numbers refer to like elements throughout. It should be noted and understood that there can be improvements and modifications made of the present invention described in detail below without departing from the scope of the invention as set forth in the accompanying claims.

Acoustic impedance (Z) is a function of a material's density and the speed of sound in/through the material. When sound travels between multiple media, differences in acoustic impedances cause at least a portion of the acoustic energy (e.g., sound waves) to be reflected at the boundary. For example, when sound travels between the body (Z=1.54 MRayl) and air (Z=0.0004 MRayl), or between water (Z=1.50 MRayl) and ceramic transducers (Z>6 MRayl), a large portion of the incident energy may not be captured by the transducer. Because so little incident energy is transmitted to the transducer, both amplification techniques and extremely sensitive transducers may be used to capture a functional signal, and energy harvesting is inefficient. Transducers that have a mismatched impedance with a medium are also more easily corrupted by external noises. To increase the amount of incident energy that can be captured by a transducer and/or to reduce noise corruption, matching layers may be used, but matching layers introduce a new set of limitations: limited bandwidth, thickness-dependent sensitivity, and bonding issues with and between matching layers.

To address these issues, the systems and methods described herein are directed to a selfpowered, impedance-matched transducer with a wide bandwidth for improved sound monitoring. The transducer may be tuned to match the acoustic impedance of the medium being monitored in the range of about 0.5 MRayls to about 5 MRayls or about 1 MRayl to about 2.5 MRayls, thereby maximizing signal transmission and removing the need for extra (e.g., matching) layers. This range of acoustic impedances covers materials such as a living body (e.g., skin), freshwater, saltwater, and most plastics.

In one application, the transducer may be specifically tailored to monitor living patients with respiratory and/or cardiovascular diseases, two of the leading causes of death globally. Placed on the body (e.g., chest) of the patient with onboard circuitry, the transducer can record lung sounds and/or heart sounds for long periods of time, which may be used to remotely monitor and diagnose patients for abnormalities without interference from speech or ambient noises. The transducer can be flexible, which allows it to be worn comfortably and deform as patients move. Low power consumption and energy harvesting enable continuous and sustainable use. The transducer may also or instead be used on low-density wood and/or underwater.

The transducer may include one or more polymer layers that may be tuned to closely match the acoustic impedance of the medium being monitored without matching layers, which minimizes the energy reflected away from the transducer while maximizing its signal to noise ratio (SNR). In addition, sources of airborne noise may be passively eliminated, as their energy is reflected at the air-transducer interface, preventing noise from contaminating the observed signal. This passive noise suppression mechanism effectively implements noise cancellation, without computationally intensive and/or expensive signal processing and noise cancellation algorithms.

With minimal (e.g., no) processing, the transducer may remove a majority of background noise at a sound level of greater than or equal to about 80 dB, while still capturing the sounds of the medium being monitored (e.g., the human chest). For example, the transducer may remove from about 97% to about 99.5% (e.g., 98.89%) of the background noise at the sound level from 71 dB to 90 dB (e.g., 85 dB), which may improve the functioning of a stethoscope. In comparison, a conventional stethoscope only achieves comparable performance at about 70 dB due to costly circuitry for noise suppression. In other words, the transducer may be tuned to the impedance of the human body to maximize the transfer of acoustic energy that is achieved with minimal interference and/or attenuation. For example, the transducer may be tuned specifically to the voice of the human body, internal sounds of the human body (e.g., the heartbeat, lungs, etc.), or a combination thereof.

The acoustic impedance matching of the transducer may be customizable. As described in greater detail below, one or more elastomers can be used as the tuned acoustic impedance polymer layer(s), which are conformable, breathable, and safe on the skin. Furthermore, the transducer can be fabricated in multiple shapes and sizes, further facilitating implementation and integration. The simplicity and straightforward fabrication of the transducer is an advantage over current transducers and allows it to be tailored to multiple applications.

Contrary to conventional transducers, the transducer described herein may use the principle of electrostatic conversion to generate an electrical response (e.g., voltage output). This is advantageous over piezoelectric and triboelectric conversion methods that have been prototyped in wearable transducers. Compared to triboelectric conversion, electrostatic conversion is less susceptible to mechanical wear and environmental conditions. Compared to piezoelectric conversion, electrostatic conversion does not involve the use of lead-based materials, is very low cost with facile processing (e.g., low temperatures, no chemicals), biocompatible, and reduces coupling between the acoustic impedance and electrical output. Voltage output is based on position and charge movement/recombination, and as such, lower frequency sounds can be observed/resolved.

The transducer may be flexible and able to conform to a variety of surfaces, complementing its customizable acoustic impedance-matching. The advent of flexible electronics and circuitry may facilitate the application of the transducer to curvilinear surfaces. The conformability of the sensor may lead to a new paradigm in monitoring sounds from the body, whereby an adhesive can be used to place the transducer on the skin and capture sounds from the voice, heart, and lungs.

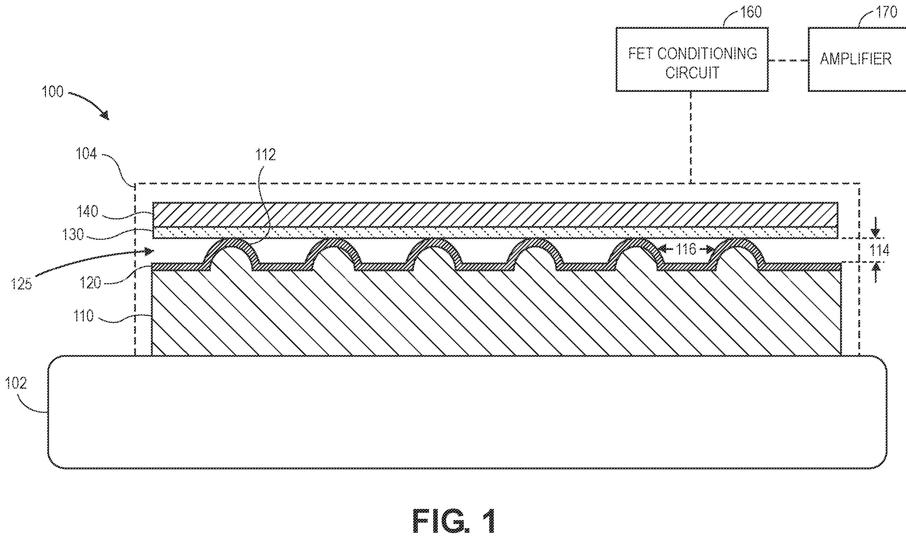

Figure 1 illustrates a schematic cross-sectional view of a transducer 100 on a medium 102, according to an embodiment. The transducer 100 may be or include an electrostatic transducer. The medium 102 may be or include a human body (e.g., a chest, neck, nose, etc.). In other embodiments, the medium 102 may be or include musical instruments, wood, water, structural members of infrastructure (e.g., buildings, bridges), aerospace materials (e.g., aluminum, titanium), and the like. In one embodiment, the transducer 100 may include a housing 104 with one or more components (e.g., layers) positioned at least partially therein. In other embodiments, the housing 104 may be omitted.

The transducer 100 may include a first (e.g., lower) layer 110 that is configured to be in direct contact with the medium 102 being monitored. The first layer 110 may be or include one or more elastomers. More particularly, the first layer 110 may be or include a tuned elastomeric matrix. For example, the first layer 110 may be or include polydimethylsiloxane (PDMS), ECOFLEX^{®}, or polyurethane. The first layer 110 may be a single layer that is fabricated and/or doped to have specific parameters to control (e.g., tune) the acoustic impedance to substantially match the impedance of the medium 102. As used herein, the impedance of the transducer 100 (e.g., the first layer 110) substantially matches the impedance of the medium 102 when a difference between the impedances is less than about 10%, about 5%, or about 1%. In one embodiment, the acoustic impedance of the transducer 100 may be controlled by varying the elastomer and/or a weight ratio of nanoparticle additives (also referred to as a dopant) that are added to the elastomer.

The nanoparticle additives may be or include ceramic, silicon dioxide, titanium dioxide, barium titanate, or a combination thereof. The concentration of the additives may be from about 1% to about 60%. For example, the concentration of the additives may be from about 1% to about 5%, about 5% to about 10%, about 10% to about 20%, about 20% to about 40%, or about 40% to about 60%.

According to the invention, the first layer 110 may be the only layer that is used/tuned to match the impedance of the medium 102. In contrast, conventional transducers may include multiple layers that are placed between the medium 102 and a surface of a sensing element. These multiple layers have different acoustic impedances and are layered so as to provide a gradient and/or gradual transition from the impedance of the medium 102 to the impedance of the sensing element. In contrast, the first layer 110 in the transducer 100 described herein provides the impedance matching and also serves as the sensing element, minimizing reflections and maximizing energy transfer. This may minimize reflections. Said another way, compared to conventional transducers with multiple matching layers, which experience a reflection at each matching layer boundary, the transducer described herein only has a single layer between the medium being monitored and the impedance matched sensing element, thereby minimizing reflections and maximizing energy transfer.

According to the invention, the first layer 1 includes one or more arrayed microstructures 112. The microstructures 112 are or include protrusions that extend away from the medium 102. The electrical and/or frequency response of the transducer 100 are controlled (e.g., tuned) by maintaining of varying the shape (e.g., hemispherical, conical, cylindrical) and/or dimensions of the microstructures 112. According to the invention, a height 114 of the microstructures 112 is from about 50 µm to about 1 mm, and a spacing 116 between two adjacent microstructures 112 is from about 50 µm to about 1 mm.

The transducer 100 includes a second layer 120 that is configured to be in direct contact with (e.g., above) the first layer 110. As shown, the second layer 120 conforms to the outer surface of the first layer 110, including the microstructures 112. The second layer 120 may be or include a thin film deposited electrode.

The transducer 100 includes a third layer 130 that is configured to be in direct contact with (e.g., above) the second layer 120. According to the invention, the third layer 130 is configured to contact the peaks of the second layer 120, but not the valleys of the second layer 120. The third layer 130 may be or include a charged electret polymer layer. An electret is a dielectric material that has a quasi-permanent electric charge or dipole polarization. An electret generates internal and external electric fields, and is the electrostatic equivalent of a permanent magnet. For example, the third layer 130 may be or include fluorinated ethylene propylene (FEP), polytetrafluoroethylene (PTFE), cyclic olefin copolymer (COC), carbon nanotubes, other polarizing materials, or a combination thereof. In one embodiment, the COC may be spin- or spray-coated and corona-charged, electrosprayed, or electrospun. In one embodiment, the FEP film may be corona-charged, which may trap charges that can be held for a large amount of time. For example, room temperature FEP has been extrapolated to hold charge for about 200 years. The electrical and/or frequency response of the transducer 100 may be controlled (e.g., tuned) by varying or maintaining the design of the third layer 130. For example, the surface area of the third layer 130 may be varied to vary the electrical response of the transducer 100.

The transducer 100 may also include a fourth (e.g., upper) layer 140 that is configured to be in direct contact with (e.g., above) the third layer 130. The fourth layer 140 may be or include a conductive (e.g., metal) backing plate. The fourth layer 140 may be separated from the third layer 130 to vary the behavior of the transducer 100. This may help the transducer 100 to harvest energy. For example, this may increase the electric field external to the third layer 130 on either side. More charges can be made available at the surface of the third layer 130 either by physical modification (e.g., patterning, incorporating porosity, etc.), by changing the material of the third layer 130, or charging parameters.

In at least one embodiment, the transducer 100 may also include an intermediate layer 125. The intermediate layer 125 may be positioned at least partially between the second and third layers 120, 130 to maintain the distances and/or deflections between the second and third layers 120, 130 at high pressures. The intermediate layer 125 may be or include a gas such as air, nitrogen, helium, or a combination thereof. The gas may be secured/trapped at least partially between the second and third layers 120, 130. In another embodiment, the intermediate layer 125 may be or include a porous polymer that is less stiff than the first layer 110. The porous polymer may prevent the microstructures 112 from collapsing, but the microstructures 112 may still deform due to the density of the porous material. The intermediate layer 125 may be used for underwater applications.

In yet another embodiment, the transducer 100 may also include another/different intermediate layer (not shown) positioned at least partially between the third and fourth layers 130, 140. This intermediate layer can be or include piezoelectric materials, which may facilitate energy harvesting.

In one embodiment, the transducer 100 may be connected to a field effect transistor (FET) conditioning circuit 160. The conditioning circuit 160 may be connected to the second layer 120 and/or the fourth layer 140 (e.g., whichever is not grounded). The conditioning circuit 160 may be configured to receive an electrical signal from the transducer 100 and to lower the impedance of the signal. In other words, the conditioning circuit 160 may act as a pre-amplifier to strengthen the signal, which may then be transmitted to an amplifier 170.

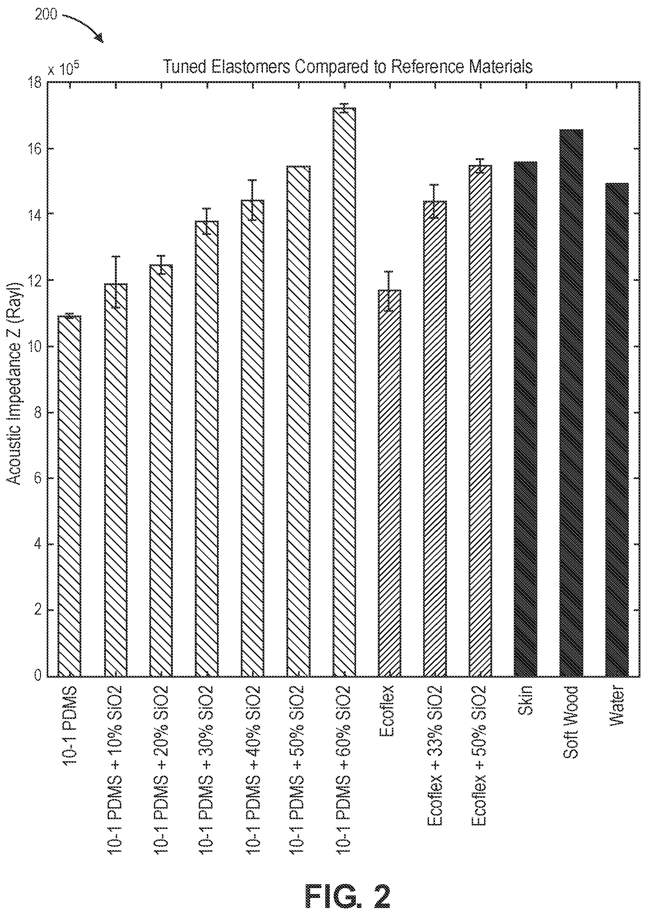

Figure 2 illustrates a graph 200 showing a range of impedance values that can be obtained by the first layer 110 (e.g., elastomer), according to an embodiment. More particularly, the graph 200 shows the acoustic impedances of two different elastomeric bases (e.g., PDMS and ECOFLEX^{®}) that are doped with varying levels of additive (e.g., silicon dioxide) nanoparticles. The PDMS has a 10-1 monomer to curing agent ratio. The PDMS is doped with 0% SiO₂, 10% SiO₂, 20% SiO₂, 30% SiO₂, 40% SiO₂, 50% SiO₂, and 60% SiO₂. The ECOFLEX^{®} is doped with 0% SiO₂, 33% SiO₂, and 50% SiO₂. In addition, three reference materials are also shown: skin, soft wood, and water. As may be seen, by selecting different materials, and varying the dopant values, any impedance value may be achieved. Although not shown, in addition to PDMS and ECOFLEX^{®}, polyurethane may also be used as the elastomeric base.

To measure the acoustic impedance, the samples are experimentally characterized in a setup including ultrasonic transducers immersed in water. The insertion technique is a relative measurement method that uses water as the reference to study the transmission of longitudinal ultrasonic waves through solid media immersed in water.

Figure 2 only includes a sampling of the possible combinations of elastomer matrices and dopants. By exploring the use of elastomers with different densities, a user may be able to further expand the range of acoustic impedance values that may be obtained. In an embodiment, the ECOFLEX^{®} may be selected due to its biocompatibility, flexibility, and ability to clean easily.

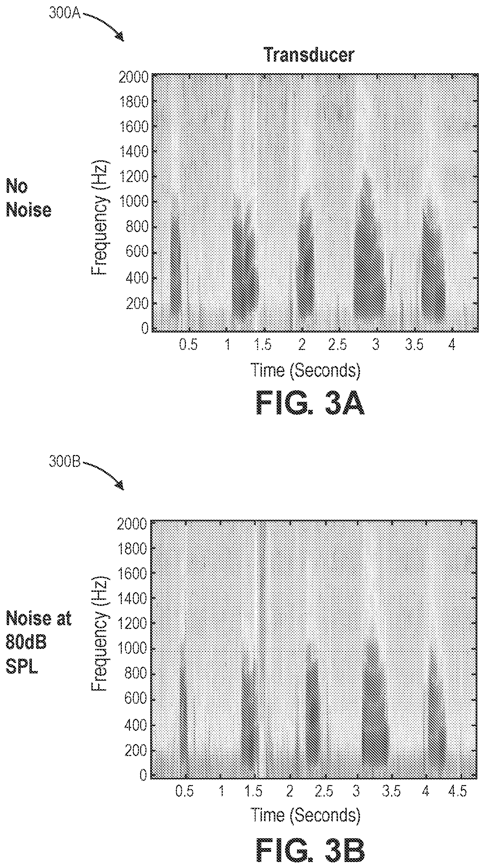

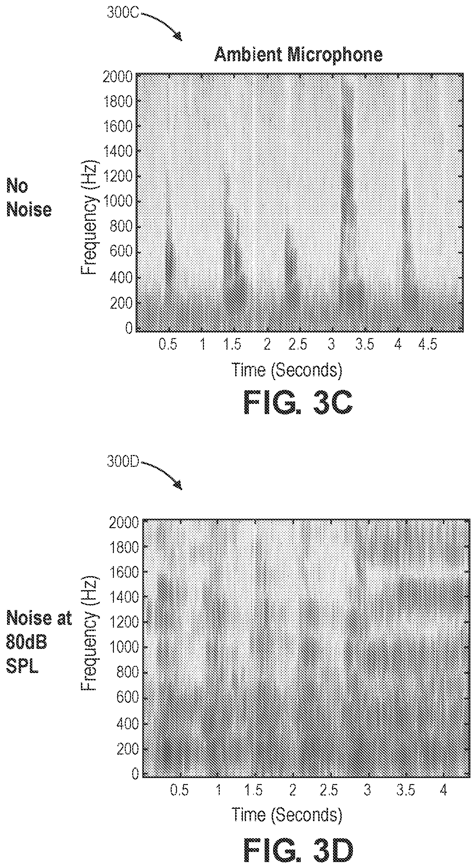

Figure 3A illustrates a graph 300A showing the acoustic output of the transducer 100 with no noise (e.g., at 45 dB SPL ambient noise), Figure 3B illustrates a graph 300B showing the acoustic output of the transducer 100 with noise (e.g., at 80 dB SPL), Figure 3C illustrates a graph 300C showing the acoustic output of an ambient microphone with no noise (e.g., at 45 dB SPL ambient noise), and Figure 3D illustrates a graph 300D showing the acoustic output of the ambient microphone with noise (e.g., at 80 dB SPL), according to an embodiment. The graphs 300A-300D are spectrograms, when counting from 6 to 10. As may be seen, the quality of acoustic recordings is maintained in highly noisy environments due to the impedance matching of the transducer 100. The transducer 100 also preserved a majority of the spectral power in the speech. The frequency response of the transducer 100 may be further optimized by changing the structure of the first layer (e.g., the elastomer 110).

The stability and stored charge may vary based on parameters including duration, temperature, and grid voltage used to charge the sample medium. The top surface of the third layer 130 may be in contact with the fourth layer 140. When the surface of the transducer 100 experiences mechanical vibration due to an acoustic wave, the first layer 110 vibrates in a similar manner. This causes the microstructures 112 on the surface of the first layer 110 to deform, creating a change in the size of the air gap between the first layer 110 and third layer 130 (e.g., the electret film layer). The air gap deformation causes a corresponding voltage output that is proportional to the original mechanical vibration.

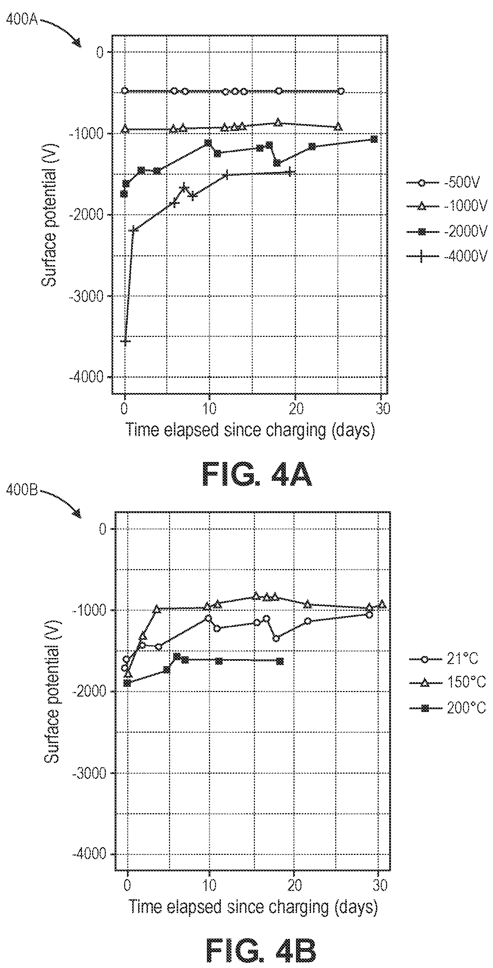

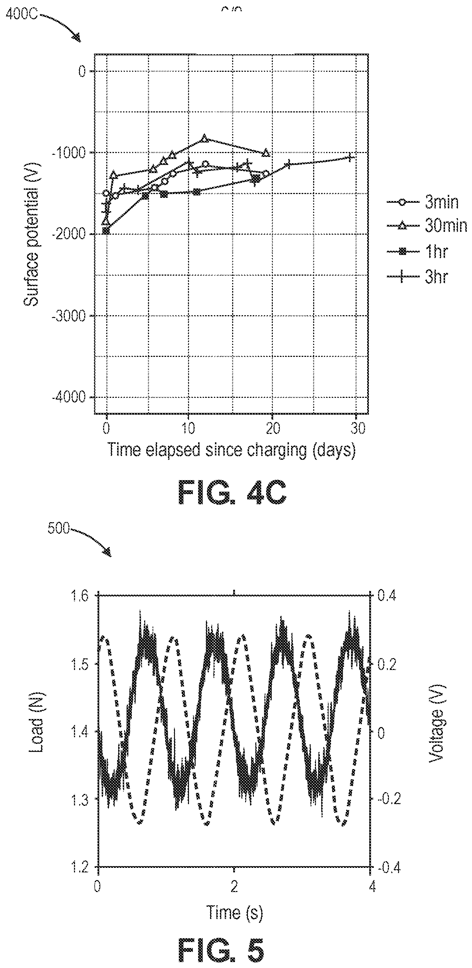

Figures 4A-4C illustrate graphs 400A-400C showing a stability of the third layer 130 of the transducer 100, according to an embodiment. More particularly, the graphs 400A-400C show the surface potential of the third layer (e.g., FEP film) 130 monitored over a period of 20-30 days. The third layer 130 in the first graph 400A is charged for 1 hour at room temperature with various grid voltages. The third layer 130 in the second graph 400B is charged for 1 hour at various temperatures with a grid voltage of 2000 V. The third layer 130 in the third graph 400C is charged for various durations at room temperature with a grid voltage of 2000 V.

The surface potential is indicative of the amount of charge stored in the third layer 130. Corona charging may be used to trap charge in the third layer 130. Corona charging applies a high voltage to a point electrode, which ionizes the air around it. The ions are accelerated toward the grounded sample, and charges are deposited. The corona charging conditions are selected to enhance both the amount and stability of charges trapped in the third layer 130. In another embodiment, the third layer 130 may be charged using an electron beam or electrospraying. The charge stability of FEP may be stable for over 100 years at room temperature.

Figure 5 illustrates a graph 500 showing examples of the voltage response of the transducer for a 1 Hz applied force, according to an embodiment. More particularly, the graph 500 shows the voltage response of the transducer 100 over time with an applied force of 0.3 N at 1 Hz and a preload of 1.3 N. The average voltage output over the time duration (e.g., 4 seconds) is 0.57 V. The size, shape, and/or spacing of the microstructures 112 can be altered to optimize the electrical response of the transducer 100.

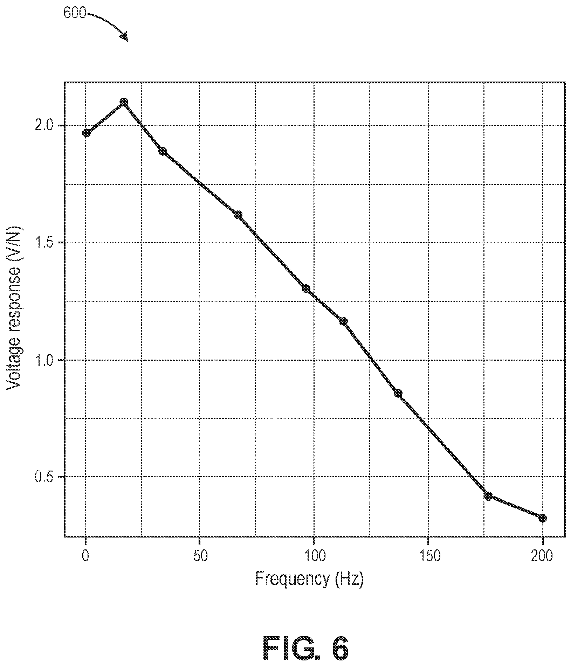

Figure 6 illustrates a graph 600 showing the voltage response of the transducer 100 as a function of frequency, according to an embodiment. The average voltage generated by the transducer 100 under mechanical loading may be measured using a long-stroke vibration exciter, a loudspeaker, or the like. Stress may be applied to one side (e.g., the first layer 110) of the transducer 100 by the exciter at a specific frequency and force. The opposite side (e.g., the fourth layer 140) of the transducer 100 may be attached to a load cell, which measures the applied force. The exciter can be adjusted to apply various preload forces to the sample medium. The data may be collected with a data acquisition system (DAQ). In the data shown in Figure 6, the voltage response is normalized by the applied force. The average preload force applied to the transducer is 1.3 N.

As may be seen, the transducer 100 is highly sensitive to low frequencies. In contrast, conventional transducers intended for airborne noise are not this sensitive. This increased sensitivity generates a high energy density in response to oceanic wave undulations, chest wall sounds and movement, material vibrations, or a combination thereof. The transducer 100 can be optimized by changing the geometry and layout (including, but not limited to, spacing and orientation) of the microstructures 112. The measurement can also be optimized by removing preloading in the setup, which compresses microstructures 112 that might be more sensitive to higher frequencies.

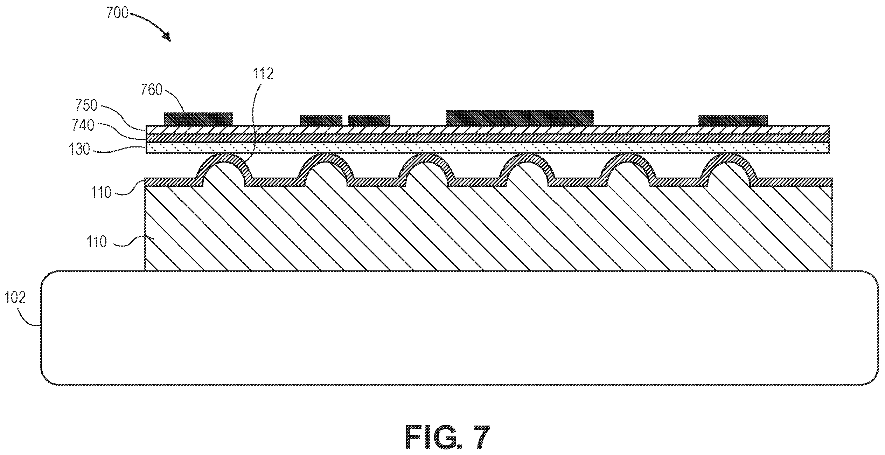

Figure 7 illustrates a schematic cross-sectional view of another transducer 700 on the medium 102, according to an embodiment. The transducer 700 may be similar to the transducer 100. For example, the transducer 700 may include the first layer 110 (e.g., including the microstructures 112), the second layer 120, and the third layer 130. However, instead of the fourth layer 140 (e.g., a conductive backing plate), the transducer 700 may instead include a fourth layer 740 positioned on (e.g., above) the third layer 130. The fourth layer 740 may be or include a thin film electrode. For example, the second layer 120 and the fourth layer 740 may both be or include thin film electrodes. In one embodiment, the fourth layer 740 may be a stiff upper electrode. In other embodiments (not shown), the fourth layer 740 may include multiple flexible upper electrodes. Using multiple flexible upper electrodes, coupled with thin-film electronics, may transform the transducer 700 into a fully conformal device.

The transducer 700 may also include a fifth layer 750 positioned on (e.g., above) the fourth layer 740. The fifth layer 750 may be or include an insulating layer (e.g., polyimide). The transducer 700 may also include a sixth layer 760 positioned on (e.g., above) the fifth layer 750. The sixth layer 760 may be or include one or more circuits combined with flexible interconnects that may provide energy, amplification, and transmission circuitry.

The transducer 100, 700 may be a flexible, biocompatible transducer that is poised to be worn by a user to improve long-term monitoring of sounds from the body and telecommunications. Continuous acoustic monitoring may be used to alert patients of abnormalities related to diseases such as heart failure (HF), pneumonia, coronavirus, COPD, or asthma. For heart failure specifically, the world's leading cause of mortality, long-term monitoring is essential to observing acute events that put patients at high risk for readmission. Approximately 22% of heart failure patients are readmitted within one month of hospital discharge. The prevalence of heart failure is projected to increase by about 46% from 2012 to 2030, and the related medical costs related are projected to increase from $21 billion to $53 billion. Recurrent hospital admissions are the main source of these costs accounting for >75% of the annual heart failure expenses. With the transducer 100, 700 described herein, a patient may be monitored for signs of congestion and acoustic signatures of pulmonary artery hypertension in the comfort of his/her home without concerns of noisy or useless data, thereby shifting the standard of care from reactive hospitalization to proactive intervention. For both cardiac and respiratory diseases, the transducer 100, 700 may streamline patient care, save millions of dollars in readmission costs, and provide at-home monitoring in times of social distancing.

Regarding telecommunications, the transducer 100, 700 may be used to improve communication in online meetings or in high-noise environments for personnel such as construction workers, engine operators, military air crews, and firefighters. The transducer 100, 700 may also facilitate communication in those environments where high sound levels are not desired and visual communication is limited, intermittent, or impossible, such as law enforcement and covert military operations.

Underwater acoustic waves are one form of communication, and one or more transducers 100, 700 may be used to facilitate this communication. Conventional underwater transducers are constructed with lead zirconate titanate (PZT), a lead-based piezoelectric ceramic material, and require processing with high voltages and temperatures to gain an electrical response. These transducers are expensive, with an average cost between $2,000 and $3,000, and are typically found in large groups of 1,000 or more that can weigh in excess of 50,000 lbs. In contrast to these conventional transducers, the transducer 100, 700 described herein is a more environmentally friendly option that uses no lead-based materials, requires little preparation to gain an electrical response, and is low-cost. The transducer 100, 700 may be well-suited for hull-mounted sonar arrays due to its flexibility, lightweight, scalability, and acoustic impedance matched to water.

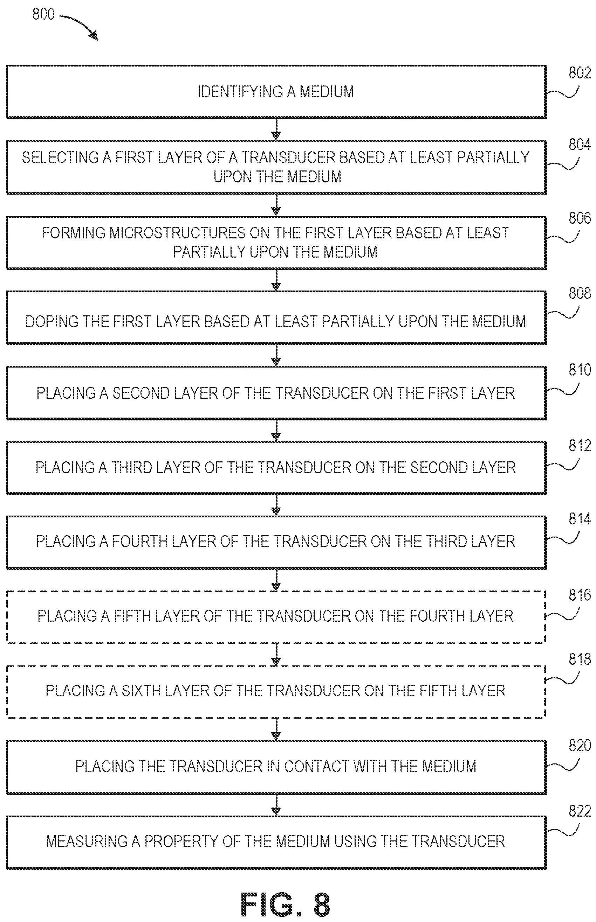

Figure 8 illustrates a flowchart of a method 800 for monitoring a medium, according to an embodiment. An illustrative order of the method 800 is provided; however, one or more steps of the method 800 may be performed in a different order, performed simultaneously, repeated, or omitted.

The method 800 may include identifying the medium 102, as at 802. As mentioned above, in one example, the medium 102 may be or include a living body. For example, the medium may be or include the chest of a living person such that the transducer 100, 700 can monitor the heart. The identified medium 102 has a particular acoustic impedance.

The method 800 may also include selecting the first layer 110 based at least partially upon the medium 102, as at 804. More particularly, this may include selecting a material to use as the first layer 110 based at least partially upon the impedance of the medium 102. Illustrative materials are described above (e.g., PDMS, ECOFLEX^{®}, or polyurethane).

The method 800 includes forming microstructures 112 on the first layer 110 based at least partially upon the medium 102, as at 806. The shape and/or dimensions of the microstructures 112 may be selected based at least partially upon the impedance of the medium 102 and desired frequency response.

The method 800 includes doping the first layer 110 based at least partially upon the medium 102, as at 808. More particularly, this may include selecting a material to use as the dopant for the first layer 110 based at least partially upon the impedance of the medium 102. Illustrative materials (e.g., additive and/or dopant) are described above (e.g., silicon dioxide nanoparticles). Doping the first layer 110 may also include selecting an amount and/or concentration of the dopant to apply to the first layer 110 based at least partially upon the impedance of the first layer 110. Examples are provided above in Figure 2.

The material of the first layer 110, the material of the dopant, the amount of the dopant, the concentration of the dopant, the size of the dopant, or a combination thereof may be selected to cause the impedance of the transducer 100, 700 (e.g., the impedance of the first layer 110) to substantially match the impedance of the medium 102.

The method 800 includes placing the second layer 120 on the first layer 110, as at 810. The second layer 120 conforms to the microstructures 112. As such, upper surface(s) of the first layer 110 and the second layer 120 have peaks and valleys.

The method 800 includes placing the third layer 130 on the second layer 120, as at 812. The third layer 130 does not conform to the microstructures 112 and the second layer 120. In other words, the third layer 130 does not have peaks and valleys. As such, a gas (e.g., air) may be trapped in the gaps between the second layer 120 and the third layer 130.

The method 800 may also include placing the fourth layer 140, 740 on the third layer 130, as at 814. The method 800 may optionally include placing the fifth layer 750 on the fourth layer 140, 740, as at 816. The method 800 may optionally include placing the sixth layer 760 on the fifth layer 750, as at 818.

One or more of the foregoing steps may be performed to build/construct the transducer 100, 700. Once built/constructed, the method 800 may include placing the transducer 100, 700 in contact with the medium 102, as at 820. More particularly, this may include placing a first (e.g., lower) side of the first layer 110 in contact with the medium 102. In one example, this may include coupling (e.g., adhering) the lower side of the first layer 110 to the chest of a living body. The microstructures 112 may be positioned on a second (e.g., upper) side of the first layer 110.

The method 800 may also include measuring a property of the medium 102 using the transducer 100, 700, as at 822. This may include converting an acoustic signal (e.g., waves) from the medium 102 into an electrical signal using the transducer 100, 700.

### Differences between transducer 100, 700 and conventional transducers

### (1) Electrostatic induction for high electrical outputs and stability in sensing applications.

Conventional acoustic impedance matched transducers use piezoelectric materials. A piezoelectric material's output is proportional to its stiffness, with a higher stiffness generally increasing acoustic impedance, but resulting in a limited acoustic response. The electrical response of piezoelectric materials that are flexible and biocompatible is also severely limited. To have a sensitive transducer with a piezoelectric material, rigid and lead-based ceramics are often used. Piezoelectret materials overcome the low sensitivity of piezoelectrics, but also have limitations including: breakdown during charging, neutralization of charges if opposite sides of void come in contact, and limited temperature stability. Triboelectric materials require separation or shearing of materials, which is difficult in thin, wearable applications. Similarly, triboelectric nanogenerators have mechanical limitations and are less reliable in real-world applications because of instability in the electrical output, such as changes with humidity. The transducer 100, 700 relies on electrostatic conversion, which is less susceptible to mechanical wear and environmental conditions. Compared to piezoelectric conversion, electrostatic conversion does not involve the use of lead-based materials, is very low cost with facile processing (e.g., low temperatures, no chemicals), biocompatible, and reduces coupling between the acoustic impedance and electrical output.

### (2) Acoustic impedance matching through elastomer doping with ceramic nanoparticles.

Conventional transducers rely on the use of conventional air-based microphones or piezoelectric materials embedded in or coupled with matching layers. These systems are limited by their design-further impedance mismatch between the transducing material and coupling material-or a set of limitations by matching layers: limited bandwidth, thickness-dependent sensitivity, and bonding issues with matching layers. These conventional systems therefore do not provide noise rejection/suppression. The transducer 100, 700 utilizes the acoustic matching layer 110 as the transducing membrane, integrating the coupling and sensing layers into a single layer for increased transmission. The use of silicone may be shaped into arbitrary 3D solids, is stable for months, even years, and is insensitive to rough handling; it is also non-toxic during preparation and application. However, the transducer 100, 700 may also or instead use ceramic nanoparticle doping to create materials with exact acoustic parameters of the intended medium. The transducer 100, 700 may also include sonic transduction in the 1 Hz to 20 kHz range.

### (3) Microstructured elastomer surface for tuning mechanical and frequency responses.

The microstructures 112 contained in the transducer 100, 700 provide restoring forces. The force magnitude can be modeled as a spring with a spring constant that is proportional to the dimensions and stiffness of the microstructures 112. As such, the size, shape, aspect ratio, and/or distribution of microstructures 112 can be adjusted to capture higher or lower frequencies. Therefore, the transducer 100, 700 can be tuned for specific frequency ranges to increase sensitivity and improve the transducer's intrinsic noise-rejection capabilities. Furthermore, multiple size, shape, aspect ratio, and/or distribution of microstructures 112 can be implemented on the same transducer 100, 700 or in an arrayed fashion, increasing the bandwidth of the transducer 100, 700. The composition of the first layer 110 can also be adjusted to further tune the frequency response, as well as to control the acoustic impedance and mechanical properties of the transducer's observation interface. The acoustic impedance of the first layer 110 can not only be matched to a specific medium, but can also be fabricated to reject interference from external noise sources, such as rejecting airborne noise when observing sounds from the human body. This degree of flexibility over the elastomer's structure and mechanical properties facilitates the broad application of the transducer 100, 700 to multiple environments.

### (4) Flexibility of the system for application to curvilinear surfaces.

The flexibility and conformability of the transducer 100, 700 is useful because of the use in fluid media and on curvilinear surfaces. Conventional transducers are limited by a rigid design following the coupling layer.

### (5) Integratable electronics.

The transducer 100, 700 may have electronics integrated directly onto the upper surface thereof. As mentioned above, amplification, energy harvesting, and transmission circuitry may be integrated into the transducer 100, 700 using stretchable interconnects.

One or more of these steps can be carried out using a non-transitory computer readable medium loaded onto a computing device such as a personal computer, tablet, phablet, smartphone, computer server, or any other computing device known to or conceivable by one of skill in the art. Indeed, any suitable hardware and software known to or conceivable by one of skill in the art could be used. The non-transitory computer readable medium can also be incorporated into the device for assessment of PAT.

A non-transitory computer readable medium is understood to mean any article of manufacture that can be read by a computer. Such non-transitory computer readable media includes, but is not limited to, magnetic media, such as a floppy disk, flexible disk, hard disk, reelto-reel tape, cartridge tape, cassette tape or cards, optical media such as CD-ROM, writable compact disc, magneto-optical media in disc, tape or card form, and paper media, such as punched cards and paper tape. The computing device can be a special computer designed specifically for this purpose. The computing device can be unique to the present invention and designed specifically to carry out the method of the present invention.

It should be noted and understood that there can be improvements and modifications made of the present invention described in detail above without departing from the scope of the invention as set forth in the accompanying claims.

## Claims

1. An acoustic transducer (100) arranged to measure mechanical vibrations of a medium (102), comprising: a first layer (110) comprising an elastomer, wherein an acoustic impedance of the first layer only is arranged to substantially match an acoustic impedance of the medium (102) that the transducer is configured to monitor; the first layer comprising a plurality of microstructures (112) on a first surface thereof, and wherein a second surface of the first layer is opposite to the first surface and is configured to be placed in contact with the medium that the transducer is configured to monitor; a second layer (120) comprising an electrode; and a third layer (130) comprising a polymer, wherein the second layer is positioned at least partially between the first layer and the third layer, and the second layer (120) substantially conforms to the microstructures such that the second layer comprises peaks and valleys, wherein the third layer is substantially flat, and a gap is present between the valleys of the second layer and the third layer; **characterised in that** a height of the microstructures (112) is from about 50 µm to about 1 mm, a spacing between two of the adjacent microstructures is from about 50 µm to about 1 mm.

2. The acoustic transducer of claim 1, wherein the elastomer comprises polydimethylsiloxane.

3. The acoustic transducer of claim 1, wherein the first layer has a monomer to curing agent ratio from about 2:1 to about 20:1.

4. The acoustic transducer of any preceding claim, wherein:
the first layer (110) is doped with a ceramic material to cause the acoustic impedance of the first layer to substantially match the acoustic impedance of the medium that the transducer is configured to monitor, and wherein the first layer is the only layer that is doped to cause the acoustic impedance to substantially match the acoustic impedance of the medium (102) that the transducer is configured to monitor; or
the first layer (110) is doped with ceramic, silicon dioxide, titanium dioxide, barium titanate, or a combination thereof to cause the acoustic impedance of the first layer to substantially match the acoustic impedance of the medium (102) that the transducer is configured to monitor, and wherein the first layer is the only layer that is doped to cause the acoustic impedance to substantially match the acoustic impedance of the medium that the transducer is configured to monitor; or
the first layer (110) is doped with between about 1% and about 60% silicon dioxide to cause the acoustic impedance of the first layer to substantially match the acoustic impedance of the medium (102) that the transducer is configured to monitor, and wherein the first layer is the only layer that is doped to cause the acoustic impedance to substantially match the acoustic impedance of the medium that the transducer is configured to monitor; or
the first layer (110) is doped with from about 1% and about 60% ceramic, silicon dioxide, titanium dioxide, barium titanate, or a combination thereof to cause the acoustic impedance of the first layer to substantially match the acoustic impedance of the medium (102).

5. The acoustic transducers of any preceding claim, wherein the microstructures are one of hemispherical, conical and cylindrical shape.

6. The acoustic transducer of any preceding claim, wherein an intermediate layer (125) of gas or a porous polymer that is less stiff than the first layer is positioned at least partially between the second and third layers.

7. The acoustic transducer of any preceding claim, wherein the polymer comprises fluorinated ethylene propylene (FEP), polytetrafluoroethylene (PTFE), or a combination thereof, or comprises a charged electret polymer.

8. The acoustic transducer of any preceding claim, further comprising a fourth layer (140) comprising a conductive metal electrode which is one of a thin film electrode, a stiff electrode, and multiple flexible electrodes, wherein the third layer is positioned at least partially between the second layer and the fourth layer.

9. The acoustic transducer of claim 8, further comprising a fifth layer (750) comprising a polyimide, wherein the fourth layer is positioned at least partially between the third layer and the fifth layer.

10. The acoustic transducer of claim 9, further comprising a sixth layer (760) comprising a circuit configured to provide energy, signal amplification, signal transmission, or a combination thereof, wherein the fifth layer is positioned at least partially between the fourth layer and the sixth layer.

11. The acoustic transducer of any preceding claim, wherein the acoustic transducer is configured to be connected to a field effect transistor (FET) conditioning circuit 160, wherein the FET conditioning circuit is configured to receive an electrical signal from the acoustic transducer and to amplify the electrical signal.

12. The acoustic transducer of claim 8, connected to a field effect transistor (FET) conditioning circuit (160) that is connected to the second layer and/or the fourth layer (140), wherein the FET conditioning circuit is configured to receive an electrical signal from the second layer and to amplify the electrical signal.

13. The acoustic transducer of claim 1, wherein the acoustic transducer is part of a stethoscope that is configured to listen to a human body, wherein the medium comprises the human body, and wherein the acoustic transducer reduces interference from noises generated in an ambient environment around the human body that corrupt signals that are output by the stethoscope.

14. A method, comprising: building an acoustic transducer (100) according to any preceding claim that is configured to measure mechanical vibrations of a medium (102), wherein building the transducer comprises: doping the first layer of the transducer with a dopant based at least partially upon the medium, wherein the material of the first layer (110), the dopant, or both cause an acoustic impedance of the first layer only to substantially match an acoustic impedance of the medium; the first layer comprising a plurality of microstructures (112) on a first surface thereof, and a second surface of the first layer is opposite to the first surface and is configured to be placed in contact with the medium that the transducer is configured to monitor; a second layer (120) comprising an electrode; and a third layer (130) comprising a polymer, wherein the second layer is positioned at least partially between the first layer and the third layer; placing a second layer (120) at least partially on the first layer so as to substantially conform to the peaks and valleys of the microstructure; and placing a third layer (130) at least partially on the second layer; placing the first layer in contact with the medium; and measuring the mechanical vibrations of the medium using the transducer.

## Patentansprüche

1. Akustischer Wandler (100), der so angeordnet ist, dass er mechanische Schwingungen eines Mediums (102) misst, umfassend:
Eine erste Schicht (110), die ein Elastomer umfasst, wobei eine akustische Impedanz nur der ersten Schicht so angeordnet ist, dass sie im Wesentlichen mit einer akustischen Impedanz des Mediums (102) übereinstimmt, das der Wandler überwachen soll; die erste Schicht umfasst eine Vielzahl von Mikrostrukturen (112) auf einer ersten Oberfläche davon, und wobei eine zweite Oberfläche der ersten Schicht der ersten Oberfläche gegenüberliegt und so konfiguriert ist, dass sie in Kontakt mit dem Medium kommt, das der Wandler überwachen soll; eine zweite Schicht (120), die eine Elektrode umfasst; und eine dritte Schicht (130), die ein Polymer umfasst, wobei die zweite Schicht mindestens teilweise zwischen der ersten Schicht und der dritten Schicht positioniert ist, und sich die zweite Schicht (120) im Wesentlichen an die Mikrostrukturen anpasst, so dass die zweite Schicht Spitzen und Täler umfasst, wobei die dritte Schicht im Wesentlichen flach ist, und eine Lücke zwischen den Tälern der zweiten Schicht und der dritten Schicht vorhanden ist,
**dadurch gekennzeichnet, dass** eine Höhe der Mikrostrukturen (112) von etwa 50 µm bis etwa 1 mm beträgt und ein Zwischenraum zwischen zwei der benachbarten Mikrostrukturen von etwa 50 µm bis etwa 1 mm beträgt.

2. Akustischer Wandler nach Anspruch 1, wobei das Elastomer Polydimethylsiloxan umfasst.

3. Akustischer Wandler nach Anspruch 1, wobei die erste Schicht ein Monomer in einem Härtungsmittel-Mischverhältnis von etwa 2:1 bis etwa 20:1 aufweist.

4. Akustischer Wandler nach einem der vorstehenden Ansprüche, wobei:
Die erste Schicht (110) mit einem Keramikmaterial dotiert ist, das bewirkt, dass die akustische Impedanz der ersten Schicht im Wesentlichen mit der akustischen Impedanz des Mediums übereinstimmt, das der Wandler überwachen soll, und wobei die erste Schicht die einzige Schicht ist, die so dotiert ist, dass sie bewirkt, dass die akustische Impedanz im Wesentlichen mit der akustischen Impedanz des Mediums (102) übereinstimmt, das der Wandler überwachen soll; oder
die erste Schicht (110) mit Keramik, Siliciumdioxid, Titandioxid, Bariumtitanat oder einer Kombination davon dotiert ist, um zu bewirken, dass die akustische Impedanz der ersten Schicht im Wesentlichen mit der akustischen Impedanz des Mediums (102) übereinstimmt, das der Wandler überwachen soll, und wobei die erste Schicht die einzige Schicht ist, die so dotiert ist, dass sie bewirkt, dass die akustische Impedanz im Wesentlichen mit der akustischen Impedanz des Mediums übereinstimmt, das der Wandler überwachen soll; oder
die erste Schicht (110) mit zwischen etwa 1% und etwa 60% Siliciumdioxid dotiert ist, um zu bewirken, dass die akustische Impedanz der ersten Schicht im Wesentlichen mit der akustischen Impedanz des Mediums (102) übereinstimmt, das der Wandler überwachen soll, und wobei die erste Schicht die einzige Schicht ist, die so dotiert ist, dass sie bewirkt, dass die akustische Impedanz im Wesentlichen mit der akustischen Impedanz des Mediums übereinstimmt, das der Wandler überwachen soll; oder
die erste Schicht (110) mit von etwa 1% bis etwa 60% Keramik, Siliciumdioxid, Titandioxid, Bariumtitanat oder einer Kombination davon dotiert ist, um zu bewirken, dass die akustische Impedanz der ersten Schicht im Wesentlichen mit der akustischen Impedanz des Mediums (102) übereinstimmt.

5. Akustischer Wandler nach einem der vorstehenden Ansprüche, wobei die Mikrostrukturen halbkugelförmig, konisch und zylinderförmig sind.

6. Akustischer Wandler nach einem der vorstehenden Ansprüche, wobei eine Zwischenschicht (125) aus Gas oder einem porösen Polymer, die weniger starr ist als die erste Schicht, mindestens teilweise zwischen der zweiten und der dritten Schicht positioniert ist.

7. Akustischer Wandler nach einem der vorstehenden Ansprüche, wobei das Polymer Fluorethylenpropylen (FEP), Polytetrafluorethylen (PTFE) oder eine Kombination davon, oder einen geladenen Elektretpolymer umfasst.

8. Akustischer Wandler nach einem der vorstehenden Ansprüche, ferner umfassend eine vierte Schicht (140), die eine leitfähige Metallelektrode umfasst, die eine aus einer Dünnfilmelektrode, einer starren Elektrode oder multiplen flexiblen Elektroden ist, wobei die dritte Schicht mindestens teilweise zwischen der zweiten Schicht und der vierten Schicht positioniert ist.

9. Akustischer Wandler nach Anspruch 8, ferner umfassend eine fünfte Schicht (750), die ein Polyimid umfasst, wobei die vierte Schicht mindestens teilweise zwischen der dritten Schicht und der fünften Schicht positioniert ist.

10. Akustischer Wandler nach Anspruch 9, ferner umfassend eine sechste Schicht (760), die einen Kreislauf umfasst, der so konfiguriert ist, dass er Energie, Signalverstärkung, Signalübertragung oder eine Kombination davon bereitstellt, wobei die fünfte Schicht mindestens teilweise zwischen der vierten Schicht und der sechsten Schicht positioniert ist.

11. Akustischer Wandler nach einem der vorstehenden Ansprüche, wobei der akustische Wandler so konfiguriert ist, dass er an eine Feldeffekttransistor (FET)-Signalaufbereitungsschaltung (160) angeschlossen ist, wobei die FET-Signalaufbereitungsschaltung konfiguriert ist, ein elektrisches Signal von dem akustischen Wandler zu empfangen und das elektrische Signal zu verstärken.

12. Akustischer Wandler nach Anspruch 8, der an eine Feldeffekttransistor (FET)-Signalaufbereitungsschaltung (160) angeschlossen ist, die mit der zweiten Schicht und/oder der vierten Schicht (140) verbunden ist, wobei die FET-Signalaufbereitungsschaltung konfiguriert ist, ein elektrisches Signal von der zweiten Schicht zu empfangen und das elektrische Signal zu verstärken.

13. Akustischer Wandler nach Anspruch 1, wobei der akustische Wandler Teil eines Stethoskops ist, das so konfiguriert ist, dass es einen menschlichen Körper abhört, wobei das Medium den menschlichen Körper umfasst und wobei der akustische Wandler die Störungen durch Geräusche reduziert, die in einer Umgebung um den menschlichen Körper herum erzeugt werden und die Signale, die von dem Stethoskop ausgegeben werden, verschlechtern.

14. Verfahren umfassend: Herstellen eines akustischen Wandlers (100) nach einem der vorstehenden Ansprüche, das so konfiguriert ist, dass es mechanische Schwingungen eines Mediums (102) misst, wobei das Herstellen des Wandlers Folgendes umfasst: Dotieren der ersten Schicht des Wandlers mit einem Dotierstoff, mindestens teilweise basierend auf dem Medium, wobei das Material der ersten Schicht (110), das Dotiermittel, oder beide, bewirken, dass eine akustische Impedanz der ersten Schicht nur im Wesentlichen mit einer akustischen Impedanz des Mediums übereinstimmt; die erste Schicht umfasst eine Vielzahl von Mikrostrukturen (112) auf einer ersten Oberfläche davon und eine zweite Oberfläche der ersten Schicht liegt der ersten Oberfläche gegenüber und ist so konfiguriert, dass sie in Kontakt mit dem Medium platziert wird, das der Wandler überwachen soll; eine zweite Schicht (120), die eine Elektrode umfasst; und eine dritte Schicht (130), die ein Polymer umfasst, wobei die zweite Schicht mindestens teilweise zwischen der ersten Schicht und der dritten Schicht positioniert ist; Platzieren einer zweiten Schicht (120) mindestens teilweise auf der ersten Schicht, um sich im Wesentlichen an die Spitzen und Täler der Mikrostruktur anzupassen; und Platzieren einer dritten Schicht (130) mindestens teilweise auf der zweiten Schicht; Platzieren der ersten Schicht in Kontakt mit dem Medium und Messen der mechanischen Schwingungen des Mediums unter Verwendung des Wandlers.

## Revendications

1. Transducteur acoustique (100) agencé de manière à mesurer les vibrations mécaniques d'un milieu (102), comprenant : une première couche (110) comprenant un élastomère, dans lequel une impédance acoustique de la première couche est agencée de manière à correspondre sensiblement à une impédance acoustique du milieu (102) que le transducteur est conçu pour surveiller ; la première couche comprenant une pluralité de microstructures (112) sur une première surface de celles-ci, et dans lequel une seconde surface de la première couche est en regard de la première surface et est conçue pour être placée en contact avec le milieu que le transducteur est conçu pour surveiller ; une deuxième couche (120) comprenant une électrode ; et une troisième couche (130) comprenant un polymère, dans lequel la deuxième couche est positionnée au moins partiellement entre la première couche et la troisième couche, et la deuxième couche (120) se conforme sensiblement aux microstructures de sorte que la deuxième couche comprend des pics et des vallées, dans lequel la troisième couche est sensiblement plate et un espace est présent entre les vallées de la deuxième couche et de la troisième couche ;
**caractérisé en ce qu'**une hauteur des microstructures (112) est comprise entre environ 50 µm et environ 1 mm, un espacement entre deux des microstructures adjacentes est compris entre environ 50 µm et environ 1 mm.

2. Transducteur acoustique selon la revendication 1, dans lequel l'élastomère comprend du polydiméthylsiloxane.

3. Transducteur acoustique selon la revendication 1, dans lequel la première couche présente un rapport monomère/agent de durcissement d'environ 2 :1 à environ 20 :1.

4. Transducteur acoustique selon une quelconque revendication précédente, dans lequel :
la première couche (110) est dopée avec un matériau céramique pour amener l'impédance acoustique de la première couche à correspondre sensiblement à l'impédance acoustique du milieu que le transducteur est conçu pour surveiller, dans lequel la première couche est la seule couche qui est dopée pour amener l'impédance acoustique à sensiblement correspondre à l'impédance acoustique du milieu (102) que le transducteur est conçu pour surveiller ; ou
la première couche (110) est dopée avec de la céramique, du dioxyde de silicium, du dioxyde de titane, du titanate de baryum, ou une combinaison de ceux-ci, pour amener l'impédance acoustique de la première couche à correspondre sensiblement à l'impédance acoustique du milieu (102) que le transducteur est conçu pour surveiller et dans lequel la première couche est la seule couche dopée pour amener l'impédance acoustique à correspondre sensiblement à l'impédance acoustique du milieu que le transducteur est conçu pour surveiller ; ou
la première couche (110) est dopée avec entre environ 1 % et environ 60 % de dioxyde de silicium pour amener l'impédance acoustique de la première couche à correspondre sensiblement à l'impédance acoustique du milieu (102) que le transducteur est conçu pour surveiller, et dans lequel la première couche est la seule couche dopée pour amener l'impédance acoustique à correspondre sensiblement à l'impédance acoustique du milieu que le transducteur est conçu pour surveiller ; ou
la première couche (110) est dopée avec entre environ 1 % et environ 60 % de céramique, de dioxyde de silicium, de dioxyde de titane, de titanate de baryum ou une combinaison de ceux-ci, pour amener l'impédance acoustique de la première couche à correspondre sensiblement à l'impédance acoustique du milieu (102).

5. Transducteur acoustique selon une quelconque revendication précédente, dans lequel les microstructures sont de forme hémisphérique, conique ou cylindrique.

6. Transducteur acoustique selon une quelconque revendication précédente, dans lequel une couche intermédiaire (125) de gaz ou d'un polymère poreux qui est moins rigide que la première couche est positionnée au moins partiellement entre les deuxième et troisième couches.

7. Transducteur acoustique selon une quelconque revendication précédente, dans lequel le polymère comprend de l'éthylène-propylène fluoré (FEP), du polytétrafluoroéthylène (PTFE), ou une combinaison de ceux-ci, ou comprend un polymère à électret chargé.

8. Transducteur acoustique selon une quelconque revendication précédente, comprenant en outre une quatrième couche (140) comprenant une électrode métallique conductrice, qui est l'une parmi une électrode à film mince, d'une électrode rigide et de multiples électrodes flexibles, dans lequel la troisième couche est positionnée au moins partiellement entre la deuxième couche et la quatrième couche.

9. Transducteur acoustique selon la revendication 8, comprenant en outre une cinquième couche (750) comprenant un polyimide, dans lequel la quatrième couche est positionnée au moins partiellement entre la troisième couche et la cinquième couche.

10. Transducteur acoustique selon la revendication 9, comprenant en outre une sixième couche (760) comprenant un circuit conçu pour fournir l'énergie, l'amplification de signal, la transmission de signal, ou une combinaison de celles-ci, dans lequel la cinquième couche est positionnée au moins partiellement entre la quatrième couche et la sixième couche.

11. Transducteur acoustique selon une quelconque revendication précédente, dans lequel le transducteur acoustique est conçu pour être connecté à un circuit de conditionnement (160) de transistor à effet de champ (FET), dans lequel le circuit de conditionnement de FET est conçu pour recevoir un signal électrique provenant du transducteur acoustique et pour amplifier le signal électrique.

12. Transducteur acoustique selon la revendication 8, connecté à un circuit de conditionnement (160) de transistor à effet de champ (FET) qui est connecté à la deuxième couche et/ou à la quatrième couche (140), dans lequel le circuit de conditionnement de FET est conçu pour recevoir un signal électrique provenant de la deuxième couche et pour amplifier le signal électrique.

13. Transducteur acoustique selon la revendication 1, dans lequel le transducteur acoustique fait partie d'un stéthoscope qui est conçu pour écouter un corps humain, dans lequel le milieu comprend le corps humain, et dans lequel le transducteur acoustique réduit les interférences provenant de bruits générés dans un environnement ambiant autour du corps humain qui corrompent les signaux qui sont émis par le stéthoscope.

14. Procédé, comprenant : la construction d'un transducteur acoustique (100) selon une quelconque revendication précédente qui est conçu pour mesurer les vibrations mécaniques d'un milieu (102), dans laquelle la construction du transducteur comprend : le dopage de la première couche du transducteur avec un dopant basé au moins partiellement sur le milieu : dans lequel le matériau de la première couche (110), le dopant, ou les deux amènent l'impédance acoustique de la première couche seulement à correspondre sensiblement à une impédance acoustique du milieu ; la première couche comprenant une pluralité de microstructures (112) sur une première surface de celles-ci et une seconde surface de la première couche est en regard de la première surface et est conçue pour être placée en contact avec le milieu que le transducteur est conçu pour surveiller ; une deuxième couche (120) comprenant une électrode ; et une troisième couche (130) comprenant un polymère, dans lequel la deuxième couche est positionnée au moins partiellement entre la première couche et la troisième couche ; le placement d'une deuxième couche (120) au moins partiellement sur la première couche de manière à épouser sensiblement les pics et les vallées de la microstructure ; et le placement d'une troisième couche (130) au moins partiellement sur la deuxième couche ; le placement de la première couche en contact avec le milieu ; et la mesure des vibrations mécaniques du milieu à l'aide du transducteur.
